# EUROPEAN PATENT SPECIFICATION

(11) **EP 2 856 170 B1**
(45) Date of publication and mention of the grant of the patent: **25.07.2018**
(21) Application number: 13729199.3
(22) Date of filing: 04.06.2013
(51) Int. Cl.: G01N 33/50, G01N 33/92, G01N 33/68

(54) **METHODS FOR DIAGNOSING CHRONIC VALVULAR DISEASE**
VERFAHREN ZUR DIAGNOSTIZIERUNG EINER CHRONISCHEN HERZKLAPPENERKRANKUNG
PROCÉDÉS PERMETTANT DE DIAGNOSTIQUER L'AFFECTION VALVULAIRE CHRONIQUE

(30) Priority: 05.06.2012 US 201261655704 P
(43) Date of publication of application: 08.04.2015
(73) Proprietor: Nestec S.A., 1800 Vevey (CH)
(72) Inventor: LI, Qinghong, Chesterfield Missouri 63305 (US); LAFLAMME, Dorothy, P., Floyd Virginia 24091 (US); HANNAH, Steven, S., Chesterfield Missouri 63017 (US)
(74) Representative: Krishnan, Sri
(86) International application number: PCT/US2013/044008
(87) International publication number: WO 2013/184628

(56) References cited:
- ARMELLE M DE LAFORCADE ET AL: "Serum Nitrate and Nitrite in Dogs with Spontaneous Cardiac Disease", J VET INTERN MED, vol. 17, no. 3, 1 May 2003 (2003-05-01), pages 315-318, XP055069815,

## Description

### CROSS REFERENCE TO RELATED APPLICATIONS

This application claims priority to U.S. Provisional Application No. 61/655704 filed June 5, 2012.

### BACKGROUND OF THE INVENTION

### Field of the Invention

The invention relates generally to methods for diagnosing chronic valvular disease and particularly to methods for diagnosing chronic valvular disease by measuring metabolites associated with chronic valvular disease.

### Description of Related Art

Cardiac disease is one of the most common disorders in animals, including animals such as dogs. Approximately 11% of dogs suffer cardiac disease, 95% of which have adult onset. One third of dogs ages 10 or over has Chronic Valvular Disease (CVD). CVD is characterized by a progressive degeneration and deformation of the atrioventricular valves, most commonly the mitral valves, resulting in early mitral valve insufficiency. This in turn leads to the appearance of a systolic heart murmur due to mitral regurgitation, wherein inadequate closure of the mitral valve causes blood to flow back to the left atrium. The affected dogs finally develop left atrioventricular volume overload, pulmonary edema, atrial dilatation, and supraventricular arrhythmias.

Although surgical or medical treatment of affected valves is possible, nutritional intervention is preferred by animal caregivers and health professionals. Early detection and treatment are imperative. However, detection can be difficult due to the lack of symptoms.

Biomarkers correlated with a particular disease or condition are useful for detecting such disease or condition when an animal is displaying minimal symptoms or asymptomatic or for diagnosing such disease or condition. In many situations, metabolites are useful biomarkers. de Laforcade et al. 2003 discloses that nitrite and nitrate, representing end products of NO metabolism, are increased in serum of dogs with chronic valvular disease as compared to healthy dogs. There is, therefore, a need for biomarkers that are useful for diagnosing chronic valvular disease in animals. Such biomarkers would enable an animal caregiver or health professional to provide the most appropriate and effective level of treatment, *e.g*., avoiding surgery when possible. Such treatment would improve the animal's quality of life.

### SUMMARY OF THE INVENTION

It is, therefore, an object of the present invention to provide methods for diagnosing chronic valvular disease in animals.

This and other objects are achieved using methods for diagnosing chronic valvular disease in an animal that involve obtaining a biological sample from the animal; analyzing the sample for the presence of one or more metabolites associated with chronic valvular disease; comparing the amount of each such metabotite identified in the sample to a corresponding amount of the same metabolite present in a sample from one or more comparable control animals that do not suffer from chronic valvular disease; and using said comparison to diagnose chronic valvular disease in the animal if the metabolites found in the animal's sample are greater than or less than the amount of the same metabolites present in the control animal's sample, depending on the particular metabolite and whether the amount of such metabolite in the sample is known to increase in animals suffering from chronic valvular disease or is known to decrease in animals suffering from chronic valvular disease.

Other and further objects, features, and advantages of the present invention will be readily apparent to those skilled in the art.

### DETAILED DESCRIPTION OF THE INVENTION

### Definitions

The term "animal" means any animal susceptible to or suffering from chronic valvular disease.

The terms "metabolite" or "biomarker" mean small molecules, the levels or intensities of which are measured in a biological sample, that may be used as markers to diagnose a disease state.

The term "comparable control animal" means an animal of the same species and type or an individual animal evaluated at two different times.

The term "diagnosing" means determining if an animal is suffering from or predicting if the animal is susceptible to developing chronic valvular disease,

As used herein, ranges are used herein in shorthand, so as to avoid having to list and describe each and every value within the range. Any appropriate value within the range can be selected, where appropriate, as the upper value, lower value, or the terminus of the range.

As used herein, the singular form of a word includes the plural, and vice versa, unless the context clearly dictates otherwise. Thus, the references "a", "an", and "the" are generally inclusive of the plurals of the respective terms. For example, reference to "a method" includes a plurality of such "methods." Similarly, the words "comprise", "comprises", and "comprising" are to be interpreted inclusively rather than exclusively. Likewise the terms "include", "including" and "or" should all be construed to be inclusive, unless such a construction is clearly prohibited from the context.

The methods and compositions and other advances disclosed here are not limited to particular methodology, protocols, and reagents described herein because, as the skilled artisan will appreciate, they may vary. Further the terminology used herein is for the purpose of describing particular embodiments only, and is not intended to, and does not, limit the scope of that which is disclosed or claimed.

Unless defined otherwise, all technical and scientific terms, terms of art, and acronyms used herein have the meanings commonly understood by one of ordinary skill in the art in the field(s) of the invention, or in the field(s) where the term is used.

All patents, patent applications, publications, technical and/or scholarly articles, and other references cited or referred to herein are in their entirety incorporated herein by reference to the extent allowed by law. The discussion of those references is intended merely to summarize the assertions made therein. No admission is made that any such patents, patent applications, publications or references, or any portion thereof, are relevant, material, or prior art. The right to challenge the accuracy and pertinence of any assertion of such patents, patent applications, publications, and other references as relevant, material, or prior art is specifically reserved.

### The Invention

In one aspect, the invention provides methods for diagnosing chronic valvular disease in an animal. Samples are analysed for the presence of one or more metabolites associated with chronic valvular disease; comparing the amount of each such metabolite identified in the sample to a corresponding amount of the same metabolite present in a sample from one or more comparable control animals that do not suffer from chronic valvular disease; and using said comparison to diagnose chronic valvular disease in the animal if the metabolites found in the animal's sample are greater than or less than the amount present in the control animal's sample. The amount or concentration of some metabolites in such samples is known to increase in animals suffering from chronic valvular disease whereas the amount or concentration of some metabolites in such samples is known to decrease in animals suffering from chronic valvular disease, The diagnosis can be made based upon only metabolites that are known to increase in amount as described, only metabolites that are known to decrease in amount as described, or a combination thereof.

In various examples, the methods comprise obtaining a biological sample from the animal; analyzing the sample for the presence of two or more metabolites associated with chronic valvular disease; comparing the amount of each such metabolite identified in the sample to a corresponding amount of the same metabolite present in a sample from one or more comparable control animals that do not suffer from chronic valvular disease; and using said comparison to diagnose chronic valvular disease in the animal if the amount of each such metabolite found in the animal's sample is less than the amount present in the control animal's sample, greater than the amount present in the control animal's sample, or a combination thereof.

The invention is based upon the discovery that the metabolites of the invention are present in the biological sample of an animal and that the amount of the metabolites in the sample serves as a biochemical indicator for diagnosing chronic valvular disease by indicating or predicting the threshold for chronic valvular disease. The invention allows caregivers and veterinary or other health care professionals to perform tests for these "biomarkers" in a sample and determine whether the animal is susceptible to or suffering from chronic valvular disease and whether there is a need for further diagnostics or treatments. Having established the need for further diagnostics or treatments, the cost and risk of such further diagnostics or treatments are justified.

In various embodiments, one or more comparable control animals that are not the animal being evaluated for chronic valvular disease and that have been determined not to suffer from chronic valvular disease are evaluated for at least one of the metabolites and the results of such evaluations are used as a baseline value for comparison with the results from an animal being evaluated for such one or more of the metabolites. In preferred embodiments, the baseline value for the metabolites is determined by evaluating numerous comparable control animals.

In other embodiments, the amount of at least one of the metabolites is determined for an animal at various times throughout the animal's life and the results used to determine if the animal is susceptible to or suffering from chronic valvular disease, *e.g*., if the amount of such at least one of the metabolites increases or decreases (as appropriate for the particular biomarker analyzed depending on whether the amount of such biomarker is known to either increase or decrease as an animal develops chronic valvular disease) as the animal ages, the animal can be diagnosed as susceptible to or suffering from chronic valvular disease. In preferred embodiments, the animal is evaluated periodically and the results for the metabolites analyzed are recorded. Then, if a subsequent evaluation shows that the amount of one or more metabolites has increased or decreased (as appropriate for the particular biomarker analyzed depending on whether the amount of such biomarker is known to either increase or decrease as an animal develops chronic valvular disease) since the last evaluation(s), the animal is diagnosed as susceptible to or suffering from chronic valvular disease.

Any biological sample containing the metabolite(s) of interest is useful in the invention. Examples include, but are not limited to, blood (serum/plasma), cerebral spinal fluid (CSF), urine, stool, breath, saliva, or biopsy of any tissue. In one embodiment, the sample is a serum sample. While the term "serum" is used herein, those skilled in the art will recognize that plasma or whole blood or a sub-fraction of whole blood may also be used.

The biological samples are analyzed for a particular metabolite using any suitable method known in the art for such metabolite. For example, and without wishing to be limiting in any manner, extracts of biological samples are amenable to analysis on essentially any mass spectrometry platform, either by direct injection or following chromatographic separation. Typical mass spectrometers are comprised of a source which ionizes molecules within the sample, and a detector for detecting the ionized molecules or fragments of molecules. Nonlimiting examples of common sources include electron impact, electrospray ionization (ESI), atmospheric pressure chemical ionization (APCI), atmospheric pressure photo ionization (APPI), matrix assisted laser desorption ionization (MALDI), surface enhanced laser desorption ionization (SELDI), and derivations thereof. Common mass separation and detection systems can include quadrupole, quadrupole ion trap, linear ion trap, time-of-flight (TOF), magnetic sector. ion cyclotron (FTMS), Orbitrap, and derivations and combinations thereof The advantage of FTMS over other MS-based platforms is its high resolving capability that allows for the separation of metabolites differing by only hundredths of a Dalton, many which would be missed by lower resolution instruments.

In preferred embodiments, the biological samples are analyzed for a selected metabolite (biomarker) using liquid chromatography mass spectrometry (LC-MS), gas chromatography mass spectrometry (GC-MS), or liquid chromatography and linear ion trap mass spectrometry when the method required is a high-throughput method.

While the use of one of the metabolites is sufficient for diagnosing chronic valvular disease, the use of one or more, two or more, three or more, or four or more of such metabolites is encompassed within the invention and may be preferred in many circumstances. The metabolites can be analyzed and used for a diagnosis in any combination.

In some examples, the diagnosis is based upon determining the amount of one or more metabolites selected from glutamate, C-glycosyltryptophan, beta-hydroxyisovalerate, oxidized glutathione, erythronate, N-acetylneuraminate, lactate, cis-aconitate, succinylcarnitine, malate, pentadecanoate (15:0), margarate (17:0), methyl palmitate (15 or 2). 12-HEPE, hexanoylcarnitine, glycerophosphorylcholine, 1-stearoylglycerophosphoinositol, N6-carbamoylthreonyladenosine, cytidine, pantothenate, N-glycolylneuraminate, X - 11400, X - 12729, X - 13422, X - 13543, X - 14272, X - 16277, 12-HETE, thromboxane B2, sarcosine (N-Methylglycine), beta-hydroxypyruvate, serine, threonine, valine, methionine, dimethylarginine (SDMA + ADMA), gamma-glutamylmethionine, glucose, 2-hydroxyoctanoate, deoxycarnitine, 1-palmitoleoylglycerolphosphocholine, 1-oleoylglycemphosphocholine, 2-oleoylglycerophosphocholine, 1-linoleoylglycerophosphocholine, 2-linoleoylglycerophosphocholine, 1-eicosadienoylglycerophosphocholine, 1-arachidonoylglycerophosphocholine, 1-docosapentaenoylglycerophosphocholine, 4-hydroxymandelate, X - 03088, X - 04357, X - 11793, X - 11818, X - 12771, X - 12786, and X - 13494.

In other examples, the diagnosis is based upon determining if the amount of each such metabolite found in the animal's sample is greater compared to the amount present in the control animal's sample, wherein the metabolites are glutamate, C-glycosyltryptophan, beta-hydroxyisovalerate, oxidized glutathione, erythronate, N-acetylneuraminate, lactate, cis-aconitate, succinylcarnitine, malate, pentadecanoate (15:0), margarate (17:0), methyl palmitate (15 or 2), 12-HEPE, hexanoylcarnitine, glycerophosphorylcholine, 1-stearoylglycerophosphoinositol, N6-carbamoylthreonyladenosine, cytidine, pantothenate, N-glycolylneuraminate, X - 11400, X - 12729, X - 13422, X - 13543, X - 14272, X - 16277, 12-HETE, and thromboxane B2. In a preferred example, the diagnosis is based upon determining if the amount of each such metabolite found in the animal's sample is greater compared to the amount present in the control animal's sample, wherein the metabolites are oxidized glutathione. N-acetylneuraminate, lactate, succinylcarnitine, hexanoylcarnitine, 12-HETE, and thromboxane B2.

In one example, the diagnosis is based upon determining if the amount of each such metabolite found in the animal's sample is less than compared to the amount present in the control animal's sample, wherein the metabolites are sarcosine (-N-Methylglycine), beta-hydroxypyruvate, serine, threonine, valine, methionine, dimethylarginine (SDMA + ADMA), gamma-glutamylmethionine, glucose, 2-hydroxyoctanoate, deoxycarnitine, 1-palmitoleoylglycerophosphocholine, 1-oleoylglycerophosphocholine, 2-oleoylglycerophosphocholine, 1-linoleoylglycerophosphocholine, 2-linoleoylglycerophosphocholine, 1-eicosadienoylglycerophosphocholine, 1-arachidonoylglycerophosphocholine, 1-docosapentaenoylglycerophosphocholine, 4-hydroxymandelate, X- 03088, X - 04357, X - 11793, X - 11818, X - 12771, X - 12786, and X - 13494. In a preferred example, the diagnosis is based upon determining if the amount of each such metabolite found in the animal's sample is less than compared to the amount present in the control animal's sample, wherein the metabolites are dimethylarginine (SDMA + ADMA), glucose, and deoxycarnitine.

In various embodiments, the animal is a canine such as a dog.

### EXAMPLES

The invention can be further illustrated by the following examples, although it will be understood that these examples are included merely for purposes of illustration and are not intended to limit the scope of the invention unless otherwise specifically indicated.

### Example 1

Study design. Serum samples were taken from two representative groups of canines. The control group (11) showed no signs of cardiac disease and the other group consisted of subjects (18) that had been previously diagnosed with cardiac disease. Samples were analyzed to obtain metabolic profiles and analyze data for biomarkers indicative of cardiac disease.

Sample Preparation. All samples were maintained at -80°C until processed. The sample preparation process was carried out using the automated MicroLab STAR® system (Hamilton Company, Reno, NV). Recovery standards were added prior to the first step in the extraction process for quality control purposes. Sample preparation was conducted using series of organic and aqueous extractions to remove the protein fraction while allowing maximum recovery of small molecules. The resulting extract was divided into two fractions; one for analysis by liquid chromatography (LC) and one for analysis by gas chromatography (GC). Samples were placed briefly on a TurboVap® (Zymark, Claiper Life Science, Hopkinton. MA) to remove the organic solvent. Each sample was then frozen and dried under vacuum. Samples were then prepared for the appropriate instrument, either LC/MS or GC/MS.

Liquid chromatography/Mass Spectrometry (LC/MS, LC/MS²): The LC/MS portion of the platform was based on a Waters ACQUITY UPLC and a Thermo-Finnigan LTQ mass spectrometer (Thermo Fisher Corporation, Waltham, MA), which consisted of an electrospray ionization (ESI) source and linear ion-trap (LIT) mass analyzer. The sample extract was split into two aliquots, dried, then reconstituted in acidic or basic LC-compatible solvents, each of which contained 11 or more injection standards at fixed concentrations. One aliquot was analyzed using acidic positive ion optimized conditions and the other using basic negative ion optimized conditions in two independent injections using separate dedicated columns. Extracts reconstituted in acidic conditions were gradient eluted using water and methanol both containing 0.1% Formic acid, while the basic extracts, which also used water/methanol, contained 6.5mM Ammonium Bicarbonate. The MS analysis alternated between MS and data-dependent MS² scans using dynamic exclusion.

Gas chromatography/Mass Spectrometry (GC/MS): The samples destined for GC/MS analysis were re-dried under vacuum desiccation for a minimum of 24 hours prior to being derivatized under dried nitrogen using bistrimethyl-silyl-triflouroacetamide (BSTFA). The GC column was 5% phenyl and the temperature ramp is from 40° to 300°C in a 16 minute period. Samples were analyzed on a Thermo-Finnigan Trace DSQ fast-scanning single-quadrupole mass spectrometer (Thermo Fisher Corporation, Waltham, MA) using electron impact ionization. The instrument was tuned and calibrated for mass resolution and mass accuracy on a daily basis. The information output from the raw data files was automatically extracted as discussed below.

Accurate Mass Determination and MS/MS fragmentation (LC/MS), (LC/MS/MS): The LC/MS portion of the platform was based on a Waters ACQUITY UPLC and a Thermo-Finnigan LTQ-FT mass spectrometer (Thermo Fisher Corporation, Waltham, MA), which had a linear ion-trap (LIT) front end and a Fourier transform ion cyclotron resonance (FT-ICR) mass spectrometer backend. For ions with counts greater than 2 million, an accurate mass measurement could be performed. Accurate mass measurements could be made on the parent ion as well as fragments. The typical mass error was less than 5 ppm. Ions with less than two million counts require a greater amount of effort to characterize. Fragmentation spectra (MS/MS) were typically generated in data dependent manner, but if necessary, targeted MS/MS could be employed, such as in the case of lower level signals.

Bioinformatics: The informatics system consisted of four major components, the Laboratory Information Management System (LIMS), the data extraction and peak-identification software, data processing tools for QC and compound identification, and a collection of information interpretation and visualization tools for use by data analysts. The hardware and software foundations for these informatics components were the LAN backbone, and a database server running Oracle 10.2.0.1 Enterprise Edition.

LIMS: The purpose of the LIMS system was to enable fully auditable laboratory automation through a secure, easy to use, and highly specialized system, The scope of the LIMS system encompasses sample accessioning, sample preparation and instrumental analysis and reporting and advanced data analysis. All of the subsequent software systems are grounded in the LIMS data structures. It has been modified to leverage and interface with the in-house information extraction and data visualization systems, as well as third party instrumentation and data analysis software.

Data Extraction and Quality Assurance: The data extraction of the raw mass spec data files yielded information that could loaded into a relational database and manipulated without resorting to BLOB manipulation. Once in the database the information was examined and appropriate QC limits were imposed. Peaks were identified using peak integration software, and component parts were stored in a separate and specifically designed complex data structure.

Compound identification: Compounds were identified by comparison to library entries of purified standards or recurrent unknown entities. Identification of known chemical entities was based on comparison to metabolomic library entries of purified standards. The combination of chromatographic properties and mass spectra gave an indication of a match to the specific compound or an isobaric entity. Additional entities could be identified by virtue of their recurrent nature (both chromatographic and mass spectral). These compounds have the potential to be identified by future acquisition of a matching purified standard or by classical structural analysis,

Results. The total number of metabolites detected in the study was 506. There were 320 named compounds and 186 unnamed compounds. The unnamed compounds represent a single molecule of discrete molecular formula and structure, but could not be matched with a currently named biochemical. Of the 506 metabolites identified, 54 were found to be statistically significant (*p*≤0.05), The statistically significant metabolites are identified in Table 1 .

**Table 1**

| Metabolites | Fold Changes (Diseased vs. Normal) |
|---|---|
| Glutamate | 1.29 |
| C-glycosyltryptophan | 132 |
| beta-hydroxyisovalerate | 1.18 |
| glutathione, oxidized (GSSG) | 2.32 |
| Etythronate | 1.25 |
| N-acetylneuraminate | 1.88 |
| Lactate | 1.32 |
| cis-aconitate | 1.30 |
| Succinylcamitine | 1.50 |
| Malate | 1.30 |
| pentadecanoate (15:0) | 1.36 |
| margarate (17:0) | 1.57 |
| methyl palmitate (15 or 2) | 1.49 |
| 12-HEPE | 1.19 |
| Hexanoylcamitine | 1.70 |
| glycerophosphorylcholine (GPC) | 1.64 |
| 1-stearoylglycerophosphoinositol | 1.57 |
| No-carbamoylthreonytadenosine | 1.25 |
| Cytidine | 1.39 |
| Pantothenate | 1.49 |
| N-glycolylneuraminate | 2.51 |
| X - 11400 | 2.96 |
| X - 12729 | 2.79 |
| X - 13422 | 2.86 |
| X - 13543 | 1.70 |
| X - 14272 | 2.45 |
| X - 16277 | 1.55 |
| 12-HETE | 2.10 |
| thromboxane B2 | 1.80 |
| sarcosine (N-Methylglycine) | 0.73 |
| beta-hydroxypyruvate | 0.81 |
| Serine | 0.84 |
| Threonine | 0.71 |
| Valine | 0.82 |
| Methionine | 0.68 |
| dimethylarginine (SDMA + ADMA) | 0.85 |
| gamma-glutamylmethionine | 0.57 |
| Glucose | 0.91 |
| 2-hydroxyoctanoate | 0.61 |
| Deoxycamitine | 0.85 |
| 1-palmitoleoylglycerophosphocholine | 0.49 |
| 1-oleoylglycerophosphocholine | 0.62 |
| 2-oleoylgycerophosphocholine | 0.42 |
| 1-linoleoylglycerophosphocholine | 0.53 |
| 2-linoleoylglycerophosphocholine | 0.48 |
| 1-eicosadienoylglycerophosphocholine | 0.60 |
| 1-arachidonoylglycerophosphocholine | 0.55 |
| 1-docosapentaenoylglycerophosphocholine | 0.45 |
| 4-hydroxymandelate | 0.61 |
| X - 03088 | 0.84 |
| X - 04357 | 0.70 |
| X - 11793 | 0.52 |
| X - 11818 | 0.51 |
| X - 12771 | 0.52 |
| X - 12786 | 0.69 |
| X - 13494 | 0.76 |

In the specification, there have been disclosed typical preferred embodiments of the invention. Although specific terms are employed, they are used in a generic and descriptive sense only and not for purposes of limitation. The scope of the invention is set forth in the claims. Obviously many modifications and variations of the invention are possible in light of the above teachings. It is therefore to be understood that within the scope of the appended claims, the invention may be practiced otherwise than as specifically described.

## Claims

1. A method for diagnosing chronic valvular disease in an animal comprising:
analysing the sample obtained from the animal for the presence of the metabolite glutamate and optionally a further metabolite associated with chronic valvular disease; and
comparing the amount of each such metabolite identified, in the sample to a corresponding amount of the same metabolite present in a sample from one or more comparable control animals that do not suffer from chronic valvular disease; and
using said comparison to diagnose chronic valvular disease in the animal if the amount of each such metabolite found in the animal's sample is greater than the amount present in the control animal's sample.

2. The method of claim 1 wherein the sample is a serum sample.

3. The method of claim 1 wherein the diagnosis is based upon determining the amount of two or more metabolites associated with chronic valvular disease.

4. The method of claim 1 wherein the diagnosis is based upon determining the amount of three or more metabolites associated with chronic valvular disease.

5. The method of claim 1 wherein the diagnosis is based upon determining the amount of four or more metabolites associated with chronic valvular disease.

6. The method, of claim 1 wherein the further metabolite is selected from the group consisting of C-glycosyltryptophan, beta- hydroxyisovalerate, oxidized glutathione, erythronate, N-acetylneuraminate, lactate, cis- aconitate, succinylcarnitine, malate, pentadecanoate (15:0), margarate (17:0), methyl palmitate (15 or 2), 12-HEPE, hexanoylcarnitine, glycerophosphorylcholine, 1-stearoylglycerophosphoinositol, N6-carbamoylthreonyladenosine, cytidine, pantothenate, N-glycolylneuraminate, 12 -HETE, thromboxane B2, sarcosine (N- Methylglycine), beta- hydroxypyruvate, serine, threonine, valine, methionine, dimethylarginine (SDMA + ADMA), gamma-glutamylmethionine glucose, 2-hydroxyoctanoate, deoxycarnitine, 1- palmitoleoylglycerophosphocholine, 1-oleoylglycerophosphocholine, 2-oleoylglycerophosphocholine, 1-linoleoylglycerophosphocholine, 2-linoleoylglycerophosphocholine, 1-eicosadienoylglycerophosphocholine, 1-arachidonoylglycerophosphocholine, 1-docosapentaenoylglycerophosphocholine, 4-hydroxymandelate.

7. The method of claim 1 wherein the animal is a canine.

8. The method of claim 1 wherein the animal is a dog.

9. A method for diagnosing chronic valvular disease in a canine comprising: analyzing a biological sample obtained from the canine for the presence of two or more metabolites associated with chronic valvular disease, wherein one of the metabolites is glutamate: and comparing the amount of each such metabolite identified in the sample to a corresponding amount of the same metabolite present in a sample from one or more comparable control canines that do not suffer from chronic valvular disease; and using said comparison to diagnose chronic valvular disease in the canine if the amount of each such metabolite found in the canine's sample is less than the amount present in the control canine's sample, wherein the metabolites are sarcosine (N- Methylglycine), beta- hydroxypyruvate, serine, threonine, valine, methionine, dimethylarginine (SDMA + ADMA), gamma-glutamylmethionine glucose, 2-hydroxyoctanoate, deoxycarnitine, 1-palmitoleoylglycerophosphocholine, 1-oleoylglycerophosphocholine, 2-oleoylglycero-phosphocholine, 1-linoleoylglycerophosphocholine, 2-linoleoylglycerophosphocholine, 1-eicosadienoylglycerophosphocholine, 1-arachidonoylglycerophosphocholine, 1-docosapentaenoylglycerophosphocholine, 4-hydroxymandelate,; and using said comparison to diagnose chronic valvular disease in the canine if the amount of each such metabolite found in the canine's sample is greater than the amount present in the control canine's sample, wherein the metabolites are glutamate, C-glycosyltryptophan, beta-hydroxyisovalerate, oxidized glutathione, erythronate, N-acetylneuraminate, lactate, cis-aconitate, succinylcarnitine, malate, pentadecanoate (15:0), margarate (17:0), methyl palmitate (15 or 2), 12-HEPE, hexanoylcarnitine, glycerophosphorylcholine, 1-stearoylglycerophosphoinositol, N6-carbamoylthreonyladenosine, cytidine, pantothenate, N-glycolylneuraminate, 12 -HETE, and thromboxane B2 or a combination thereof.

## Patentansprüche

1. Verfahren zum Diagnostizieren von chronischer Klappenerkrankung bei einem Tier, umfassend:
Analysieren der von dem Tier erhaltenen Probe auf Anwesenheit des Metaboliten Glutamat und wahlweise eines weiteren Metaboliten, der mit chronischer Klappenerkrankung assoziiert ist; und
Vergleichen der Menge jedes Metaboliten, der in der Probe festgestellt wird, mit einer entsprechenden Menge desselben Metaboliten, der in einer Probe von einem oder mehreren vergleichbaren Kontrolltieren festgestellt wird, die nicht an chronischer Klappenerkrankung leiden; und
Verwenden des Vergleichs zum Diagnostizieren von chronischer Klappenerkrankung bei dem Tier, wenn die Menge jedes Metaboliten, die in der Probe des Tiers festgestellt wurde, größer ist als die Menge, die in der Probe von dem Kontrolltier vorliegt.

2. Verfahren nach Anspruch 1, wobei es sich bei der Probe um eine Serumprobe handelt.

3. Verfahren nach Anspruch 1, wobei die Diagnose auf dem Bestimmen der Menge von zwei oder mehr Metaboliten basiert, die mit chronischer Klappenerkrankung assoziiert sind.

4. Verfahren nach Anspruch 1, wobei die Diagnose auf dem Bestimmen der Menge von drei oder mehr Metaboliten basiert, die mit chronischer Klappenerkrankung assoziiert sind.

5. Verfahren nach Anspruch 1, wobei die Diagnose auf dem Bestimmen der Menge von vier oder mehr Metaboliten basiert, die mit chronischer Klappenerkrankung assoziiert sind.

6. Verfahren nach Anspruch 1, wobei der weitere Metabolit ausgewählt ist aus der Gruppe bestehend aus C-Glycosyltryptophan, beta-Hydroxyisovalerat, oxidiertes Glutathion, Erythronat, N-Acetylneuraminat, Lactat, cis-Aconitat, Succinylcarnitin, Malat, Pentadecanoat (15:0), Margarat (17:0), Methylpalmitat (15 oder 2), 12-HEPE, Hexanoylcarnitin, Glycerophosphorylcholin, 1-Stearoylglycerophosphoinositol, N6-Carbamoylthreonyladenosin, Cytidin, Pantothenat, N-Glycolylneuraminat, 12-HETE, Thromboxan B2, Sarcosin (N-Methylglycin), beta-Hydroxypyruvat, Serin, Threonin, Valin, Methionin, Dimethylarginin (SDMA + ADMA), gamma-Glutamylmethion, Glucose, 2-Hydroxyoctanoat, Deoxycarnitin, 1-Palmitoleoylglycerophosphocholin, 1-Oeoylglycerophosphocholin, 2-Oeoylglycerophosphocholin, 1-Linoleoylglycerophosphocholin, 2-Linoleoylglycerophosphocholin, 1-Eicosadienoylglycerophosphocholin, 1-Arachidonoylglycerophosphocholin, 1-Docosapentaenoylglycerophosphocholin, 4-Hydroxymandelat.

7. Verfahren nach Anspruch 1, wobei das Tier ein Hund ist.

8. Verfahren nach Anspruch 1, wobei das Tier ein Haushund ist.

9. Verfahren zum Diagnostizieren von chronischer Klappenerkrankung bei einem Hund, umfassend:
Analysieren einer biologischen, von einem Hund erhaltenen Probe auf die Anwesenheit von zwei oder mehr Metaboliten, die mit chronischer Klappenerkrankung assoziiert sind, wobei einer der Metaboliten Glutamat ist; und
Vergleichen der Menge jedes Metaboliten, der in der Probe festgestellt wird, mit einer entsprechenden Menge desselben Metaboliten, der in der Probe von einem oder mehreren vergleichbaren Kontrollhunden festgestellt wird, die nicht an einer chronischen Klappenerkrankung leiden; und
Verwenden des Vergleichs zum Diagnostizieren von chronischer Klappenerkrankung bei einem Hund, wenn die Menge jedes Metaboliten, der in der Hundeprobe festgestellt wurde, geringer ist als die Menge, die in der Probe von dem Kontrollhund vorliegt, wobei die Metaboliten Sarcosin (N-Methylglycin), beta-Hydroxypyruvat, Serin, Threonin, Valin, Methionin, Dimethylarginin (SDMA + ADMA), gamma-Glutamylmethion, Glucose, 2-Hydroxyoctanoat, Deoxycarnitin, 1-Palmitoleoylglycerophosphocholin, 1-Oleoylglycerophosphocholin, 2-Oleoylglycerophosphocholin, 1-Linoleoylglycerophosphocholin, 2-Linoleoylglycerophosphocholin, 1-Eicosadienoylglycerophosphocholin, 1-Arachidonoylglycerophosphocholin, 1-Docosapentaenoylglycerophosphocholin, 4-Hydroxymandelat sind;
und Verwenden des Vergleichs zum Diagnostizieren von chronischer Klappenerkrankung bei dem Hund, wenn die Menge jedes der Metaboliten, die in der Hundeprobe festgestellt wurden, größer ist als die Menge, die in der Probe von dem Kontrollhund vorliegt, wobei die Metaboliten Glutamat, C-Glycosyltryptophan, beta-Hydroxyisovalerat, oxidiertes Glutathion, Erythronat, N-Acetylneuraminat, Lactat, cis-Aconitat, Succinylcarnitin, Malat, Pentadecanoat (15:0), Margarat (17:0), Methylpalmitat (15 oder 2), 12-HEPE, Hexanoylcarnitin, Glycerophosphorylcholin, 1-Stearoylglycerophosphoinositol, N6-Carbamoylthreonyladenosin, Cytidin, Pantothenat, N-Glycolylneuraminat, 12-HETE und Thromboxan B2 oder eine Kombination davon sind.

## Revendications

1. Méthode de diagnostic d'une valvulopathie chronique chez un animal, comprenant :
l'analyse de l'échantillon obtenu auprès de l'animal pour déceler la présence du métabolite glutamate et éventuellement d'un métabolite supplémentaire associé à la valvulopathie chronique ; et
la comparaison de la quantité de chaque tel métabolite identifiée dans l'échantillon à une quantité correspondante du même métabolite présente dans un échantillon provenant d'un ou plusieurs animaux témoins comparables qui ne souffrent pas de valvulopathie chronique ; et
l'utilisation de ladite comparaison pour diagnostiquer une valvulopathie chronique chez l'animal si la quantité de chaque tel métabolite trouvée dans l'échantillon de l'animal est supérieure à la quantité présente dans l'échantillon de l'animal témoin.

2. Méthode selon la revendication 1, dans laquelle l'échantillon est un échantillon de sérum.

3. Méthode selon la revendication 1, dans laquelle le diagnostic est fondé sur la détermination de la quantité de deux métabolites ou plus associés à une valvulopathie chronique.

4. Méthode selon la revendication 1, dans laquelle le diagnostic est fondé sur la détermination de la quantité de trois métabolites ou plus associés à une valvulopathie chronique.

5. Méthode selon la revendication 1, dans laquelle le diagnostic est fondé sur la détermination de la quantité de quatre métabolites ou plus associés à une valvulopathie chronique.

6. Méthode selon la revendication 1, dans laquelle le métabolite supplémentaire est choisi dans le groupe constitué de C-glycosyltryptophane, bêta-hydroxy-isovalérate, glutathion oxydé, érythronate, N-acétylneuraminate, lactate, cis-aconitate, succinylcarnitine, malate, pentadécanoate (15:0), margarate (17:0), palmitate de méthyle (15 ou 2), 12-HEPE, hexanoylcarnitine, glycérophosphorylcholine, 1-stéaroylglycérophospho-inositol, N6-carbamoylthréonyladénosine, cytidine, pantothénate, N-glycolylneuraminate, 12-HETE, thromboxane B2, sarcosine (N-méthylglycine), bêta-hydroxypyruvate, sérine, thréonine, valine, méthionine, diméthylarginine (SDMA + ADMA), gamma-glutamylméthione, glucose, 2-hydroxyoctanoate, désoxycarnitine, 1-palmitoléoylglycérophosphocholine, 1-oléoylglycérophosphocholine, 2-oléoylglycérophosphocholine, 1-linoléoylglycérophosphocholine, 2-linoléoylglycérophosphocholine, 1-eicosadiénoylglycérophosphocholine, 1-arachidonoylglycérophosphocholine, 1-docosapenta-énoylglycérophosphocholine, 4-hydroxymandélate.

7. Méthode selon la revendication 1, dans laquelle l'animal est un canidé.

8. Méthode selon la revendication 1, dans laquelle l'animal est un chien.

9. Méthode de diagnostic d'une valvulopathie chronique chez un canidé, comprenant :
l'analyse d'un échantillon biologique obtenu auprès du canidé pour déceler la présence de deux métabolites ou plus associés à une valvulopathie chronique, dans laquelle un des métabolites est le glutamate : et
la comparaison de la quantité de chaque tel métabolite identifiée dans l'échantillon à une quantité correspondante du même métabolite présente dans un échantillon provenant d'un ou plusieurs canidés témoins comparables qui ne souffrent pas de valvulopathie chronique ; et
l'utilisation de ladite comparaison pour diagnostiquer une valvulopathie chronique chez le canidé si la quantité de chaque tel métabolite trouvée dans l'échantillon du canidé est inférieure à la quantité présente dans l'échantillon du canidé témoin, dans laquelle les métabolites sont la sarcosine (N-méthylglycine), le bêta-hydroxypyruvate, la sérine, la thréonine, la valine, la méthionine, la diméthylarginine (SDMA + ADMA), la gamma-glutamylméthione, le glucose, le 2-hydroxyoctanoate, la désoxycarnitine, la 1-palmitoléoylglycérophosphocholine, la 1-oléoylglycérophosphocholine, la 2-oléoylglycérophosphocholine, la 1-linoléoylglycérophosphocholine, la 2-linoléoylglycérophosphocholine, la 1-eicosadiénoylglycérophosphocholine, la 1-arachidonoylglycérophosphocholine, la 1-docosapenta-énoylglycérophosphocholine, le 4-hydroxymandélate ;
et l'utilisation de ladite comparaison pour diagnostiquer une valvulopathie chronique chez le canidé si la quantité de chaque tel métabolite trouvée dans l'échantillon du canidé est supérieure à la quantité présente dans l'échantillon du canidé témoin, dans laquelle les métabolites sont le glutamate, le C-glycosyltryptophane, le bêta-hydroxy-isovalérate, le glutathion oxydé, l'érythronate, le N-acétylneuraminate, le lactate, le cis-aconitate, la succinylcarnitine, le malate, le pentadécanoate (15:0), le margarate (17:0), le palmitate de méthyle (15 ou 2), le 12-HEPE, l'hexanoylcarnitine, la glycérophosphorylcholine, le 1-stéaroylglycérophospho-inositol, la N6-carbamoylthréonyladénosine, la cytidine, le pantothénate, le N-glycolylneuraminate, le 12-HETE et le thromboxane B2 ou une combinaison de ceux-ci.
